# EUROPEAN PATENT SPECIFICATION

(11) **EP 1 133 315 B1**
(45) Date of publication and mention of the grant of the patent: **01.02.2006**
(21) Application number: 99965880.0
(22) Date of filing: 23.11.1999
(51) Int. Cl.: A61K 39/395, G01N 33/53, A61P 1/04, A61P 11/06, A61P 19/02, A61P 37/08, A61P 37/00

(54) **ANTAGONISTS OF THE ALPHA E BETA 7 INTEGRIN AS THERAPEUTIC AGENTS FOR INFLAMMATORY DISEASES**
ANTAGONISTE DES ALPHA-E-BETA-7 INTEGRINS ALS THERAPEUTISCHE AGENZIEN GEGEN ENTZÜNDUNGSKRANKHEITEN
ANTAGONISTES DE L'INTEGRINE ALPHA E BETA 7 UTILISES COMME AGENTS THERAPEUTIQUES POUR TRAITER LES MALADIES INFLAMMATOIRES

(30) Priority: 25.11.1998 US 109957 P
(43) Date of publication of application: 19.09.2001
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: LUDVIKSSON, Bjorn, R., 220 Hafnarfjorfur (IS); STROBER, Warren, Bethesda, MD 20817 (US); EHRHARDT, Rolf, O., San Francisco, CA 94114 (US)
(74) Representative: Leissler-Gerstl, Gabriele
(86) International application number: PCT/US1999/027817
(87) International publication number: WO 2000/030681

(56) References cited:
- WO-A-95/22610
- WO-A-95/29693
- WO-A-97/25423
- WO-A-98/06248
- C. JORGENSEN ET AL.: "Inhibition of human mucosal lymphocytes migration in rheumatoid synovium engrafted in SCID mice by antibodies against LFA-1 and alphaEbeta7 adhesion molecules." ARTHRITIS AND RHEUMATISM, vol. 37, no. 9 suppl., September 1994 (1994-09), page S220 XP002129083 New York, NY, USA
- C. JORGENSEN ET AL.: "Overexpression of integrin alphaEbeta7 on synovial fluid lymphocytes in rheumatoid arthritis and its regulation by sulfasalazine, sulfapyridine, 5-ASA and methotrexate." ARTHRITIS AND RHEUMATISM, vol. 37, no. 9 suppl., September 1994 (1994-09), page S255 XP000867268 New York, NY, USA
- M. PANG ET AL.: "Up-regulation of alphaEbeta7, a novel integrin adhesion molecule, on T cells from systemic lupus erythematosus patients with specific epithelial involvement." ARTHRITIS AND RHEUMATISM, vol. 41, no. 8, August 1998 (1998-08), pages 1456-1463, XP000867228 New York, NY, USA
- E. ROSTAPSHOVA ET AL.: "Integrin-mediated interactions influence the tissue specificity of CD8+ cytolytic T lymphocytes." EUROPEAN JOURNAL OF IMMUNOLOGY, vol. 28, no. 10, October 1998 (1998-10), pages 3031-3039, XP000867236 Weinheim, Germany
- G. HADLEY ET AL.: "The epithelial cell-specific integrin, CD103 (alphaE integrin), defines a novel subset of alloreactive CD8+ CTL." THE JOURNAL OF IMMUNOLOGY, vol. 159, no. 8, 15 October 1997 (1997-10-15), pages 3748-3756, XP002129084 Baltimore, MD, USA
- G. RUSSELL ET AL.: "Distinct structural and functional epitopes of the alphaEbeta7 integrin." EUROPEAN JOURNAL OF IMMUNOLOGY, vol. 24, 1994, pages 2832-2841, XP000672907 Weinheim, Germany
- DATABASE WPI Week 9503 Derwent Publications Ltd., London, GB; AN 1995-018280 XP002129087 & JP 06 303990 A (KANEBO LTD.), 1 November 1994 (1994-11-01)
- D. ALBERT ET AL.: "Gastrointestinal inflammatory disease in SIV-smmPBj-infected Macaca nemestrina is E-selectin independent yet characterized by recruitment of nondividing (KI-67), mucosal-specific (alphaEbeta7+) lymphocytes." JOURNAL OF MEDICAL PRIMATOLOGY, vol. 23, no. 4, June 1994 (1994-06), page 241 XP000867225 Copenhagen, Denmark
- D. ELEWAUT ET AL.: "Enrichment of T cells carrying beta7 integrins in inflamed synovial tissue from patients with early spondyloarthropathy, compared to rheumatoid arthritis." JOURNAL OF RHEUMATOLOGY, vol. 25, no. 10, October 1998 (1998-10), pages 1932-1937, XP000867226 Toronto, Canada
- D. ANDREW ET AL.: "Distribution of alpha4beta7 and alphaEbeta7 integrins on thymocytes, intestinal epithelial lymphocytes and peripheral lymphocytes." EUROPEAN JOURNAL OF IMMUNOLOGY, vol. 26, no. 4, April 1996 (1996-04), pages 897-905, XP000867271 Weinheim, Germany
- M. BAUMGART ET AL.: "Increase in the expression of alphaEbeta7, characteristic of intestinal intraepithelial lymphocytes, on T cells in the lung epithelium of patients with interstitial lung diseases and in synovial fluid of patients with rheumatic diseases." IMMUNOBIOLOGY, vol. 196, no. 4, December 1996 (1996-12), pages 415-424, XP000867231 Stuttgart, Germany
- C. ELANGBAM ET AL.: "Cell adhesion molecules-Update." VETERINARY PATHOLOGY, vol. 34, no. 1, January 1997 (1997-01), pages 61-73, XP000867246 Basel, Switzerland
- K. CEPEK ET AL.: "Integrin alphaEbeta7 mediates adhesion of T lymphocytes to epithelial cells." THE JOURNAL OF IMMUNOLOGY, vol. 150, no. 8 part 1, 15 April 1993 (1993-04-15), pages 3459-3470, XP002129085 Baltimore, MD, USA
- B. LUDVIKSSON ET AL.: "Administration of mAb against alphaEbeta7 prevents and ameliorates immunization-induced colitis in IL-2-/- mice." THE JOURNAL OF IMMUNOLOGY, vol. 162, no. 8, 15 April 1999 (1999-04-15), pages 4975-4982, XP002129086 Baltimore, MD, USA
- P. KILSHAW: "AlphaEbeta7." MOLECULAR PATHOLOGY, vol. 52, no. 4, August 1999 (1999-08), pages 203-207, XP000867245 London, GB

## Description

### BACKGROUND OF THE INVENTION

### FIELD OF THE INVENTION

The present invention relates to the use of an α^{E}β₇ antibody for treating or preventing inflammatory bowel disease. Further provided is a method of screening substances effective in reducing and preventing the inflammatory effects of α^{E}β₇ in inflammatory bowel disease.

### BACKGROUND ART

The pathogenesis of autoimmune inflammatory reactions, both those occurring in humans and those occurring in various murine models of autoimmunity may depend on the traffic of lymphocytes from sites of induction to sites of inflammation (1-4). Such traffic is now known to be mediated, at least in part, by an interaction between the integrin, α₄β₇, on circulating lymphocytes and the addressin MAdCAM-1 on endothelial cells (5-10). The "homing" interactions governing the entry of cells into tissues are accompanied by additional adhesion molecule-ligand interactions that ensure the retention of cells in the target tissue (11).

In recent studies, it has been shown that IL-2 -/- mice do not develop significant colitis when maintained in a pathogen-free environment, but can be induced to develop severe colitis rapidly and predictively following i.p. injection of TNP-substituted protein in complete Freund's adjuvant (12). Such induced colitis is a Thl CD4 lymphocyte-driven inflammation that is similar to the spontaneous colitis developed in IL-2 -/- mice maintained in a conventional environment (12-14).

The role of α^{E}β₇ in this experimental model of chronic intestinal inflammation was analyzed in the IL-2 -/- model. The present invention shows that administration of anti-α^{E}β₇ prevents colonic inflammation and that anti-α^{E}β₇ reverses pre-existing inflammation.

WO 95/29693 discloses methods and compositions for modulating heterotypic E-cadherin interactions with T-lymphocytes wherein the interactions between αEβ7 and E-cadherin can be inhibited by antibodies directed against E-cadherin. Thereby the adhesion between an IEL and E-cadherin is inhibited.

C. Jorgensen et al, ARTHRITIS AND RHEUMATISM, vol. 37, No. 9, suppl., September 1994 (1994-09), page S220 describe the inhibition of human mucosal lymphocytes migration in rheumatoid synovium engrafted in SCID mice by antibodies against αEβ7 adhesion molecules. From this document no conclusion could be drawn to the treatment of inflammatory bowel disease which is manifested in the lamina propria.

The present invention provides a novel method to prevent and treat inflammatory reactions in autoimmune disease, allergic disease, graft-versus-host disease.and transplantation rejection by administering antagonists of α^{E}β₇ integrin. The present invention further provides a method for screening for substances effective in their ability to inhibit the inflammatory effects of α^{E}β₇ integrin.

### SUMMARY OF THE INVENTION

The present invention provides the use of an α^{E}β₇, antibody as defined in claim 1. The antibody can be used in a method of preventing inflammation in a subject, as an antagonist to α^{E}β₇ in the treatment of inflammatory bowel disease.

Also provided is a method of screening for a substance effective in reducing the inflammatory effects of α^{E}β₇ in inflammatory bowel disease comprising administering the substance to a non-human animal having an established inflammatory disease; assaying inflammatory tissue cells from the animal for an amount of secretion of proinflammatory cytokines, inflammatory cytokines or chemokines, whereby a decrease in the amount of proinflammatory cytokines, inflammatory cytokines or chemokines secreted by the inflammatory tissue cells of the animal as compared to the amount of proinflammatory cytokines, inflammatory cytokines or chemokines secreted by inflammatory tissue cells of a control animal having an inflammatory disease and without having the substance administered indicates the substance is effective in reducing the inflammatory effect of αEβ7. This method further comprises the step of evaluating the effect of the antagonist of αEβ7 in reducing the adhesion/retention/influx of αEβ7+ inflammatory cells into the inflammatory tissue., a reduction indicating that the antagonist is acting at the level of αEβ7.

Additionally, the present invention provides a method of screening for a substance effective in preventing the inflammatory effects of α^{E}β₇, comprising administering the substance to an animal susceptible to an inflammatory disease; subjecting the animal to treatment that will induce an inflammatory response; assaying inflammatory tissue cells from the animal for an amount of secretion of proinflammatory cytokines, inflammatory cytokines or chemokines, whereby a lack of increase in the amount of proinflammatory cytokines, inflammatory cytokines or chemokines secreted by the inflammatory tissue cells of the animal as compared to an increase in the amount of proinflammatory cytokines, inflammatory cytokines or chemokines secreted by inflammatory tissue cells of a control animal having an inflammatory disease and without having the substance administered indicates the substance is effective in preventing the inflammatory disease by inhibiting the inflammatory effect of α^{E}β₇. This method further comprises the step of evaluating the effect of the antagonist of αEβ7 in reducing the adhesion/retention/influx of αEβ7+ inflammatory cells into the inflammatory tissue. In both cases the inflammatory diseases is inflammatory bowel disease.

### BRIEF DESCRIPTION OF THE DRAWINGS

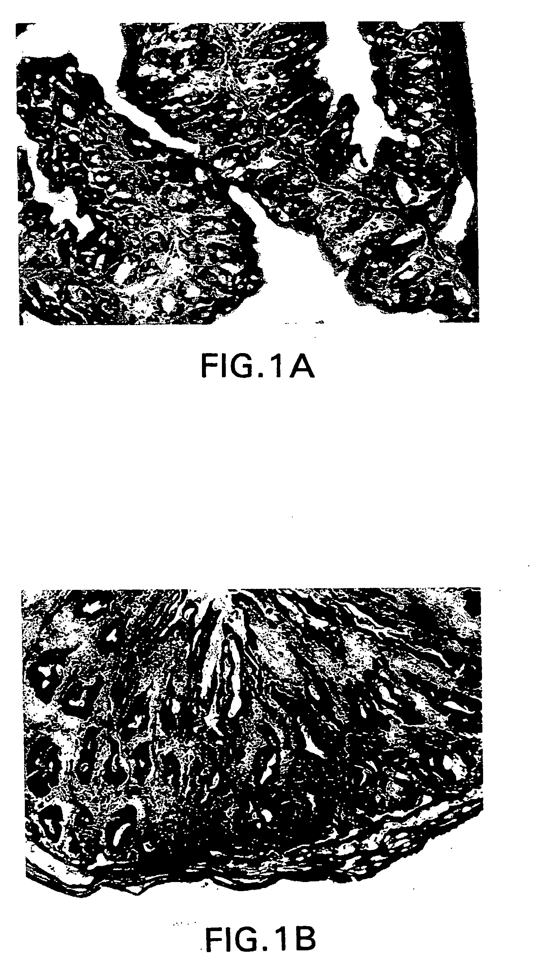

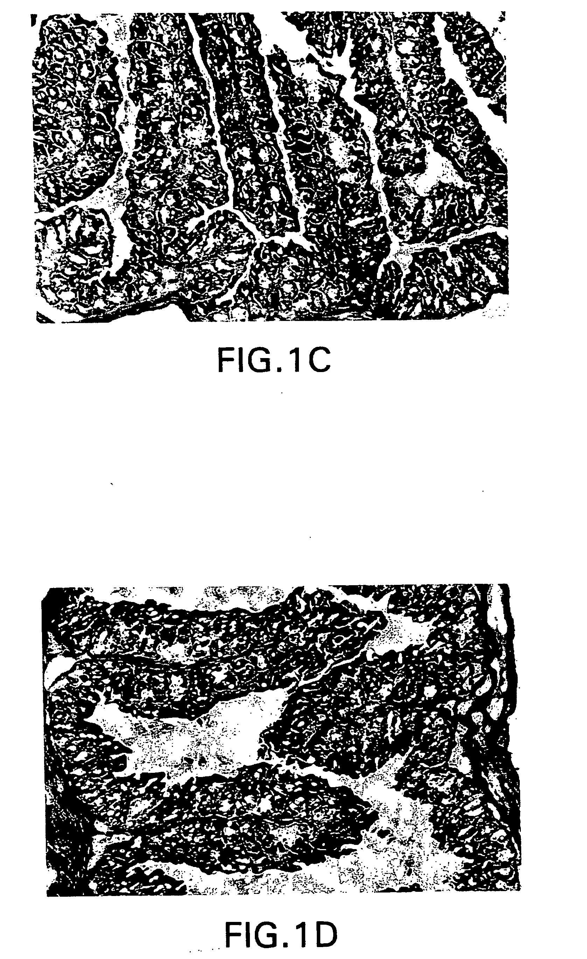

Figure 1. Administration of anti-α^{E}β₇ mAb prevents and ameliorates TNP-OVA induced colitis in IL-2-/- mice. Photomicrographs from hematoxylin/eosin-stained colonic tissue. A, normal colonic tissue from mouse that was injected with PBS and rat IgG2a. B, significant mononuclear cell inflammation seen in colonic mucosa of a IL-2-/- mouse immunized with TNP-OVA in CFA and co-injected with rat IgG2a. C and D, near-normal appearance of the colonic mucosa following either preventive or treatment regimen of anti-α^{E}β₇ mAb in mice injected with TNP-OVA in CFA, respectively. 100X magnification. Results represent data obtained from 3-4 mice in each group.

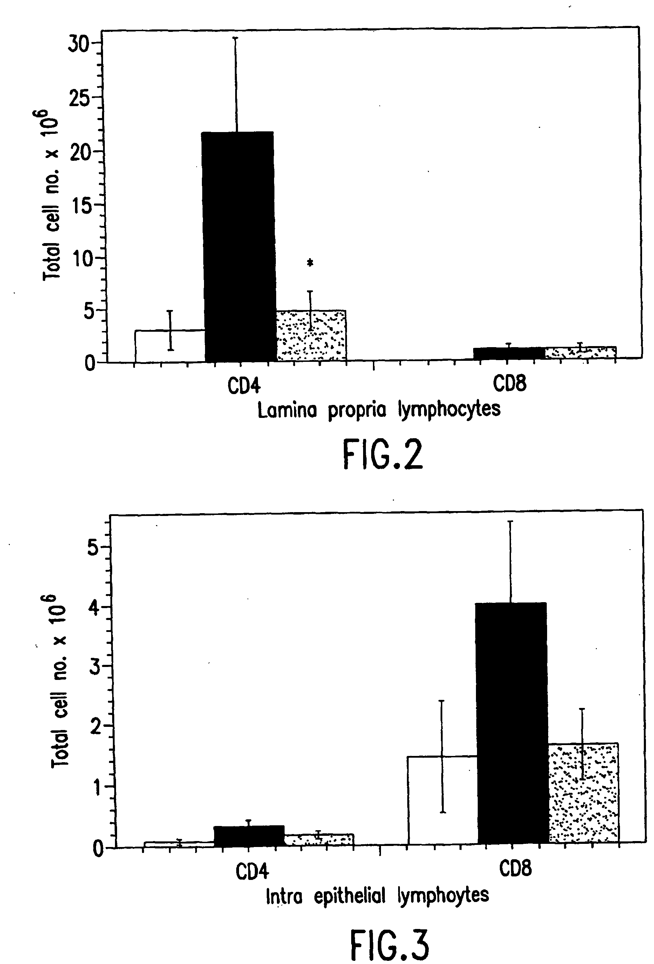

Figure 2. Decreased number of CD4+ LPL following treatment with anti-α^{E}β₇ mAb. LPL were isolated, counted and analyzed for CD4+ and CD8+ phenotype by flow cytometry (see Examples). Total no. of LPL was analyzed from IL-2-/- mice that were injected with PBS + Rat IgG2a (n=3; white bars), TNP-OVA in CFA + Rat IgG2a (n=4; black bars) and TNP-OVA in CFA + anti-aEb7 (n=4; dotted bars). *p < 0.01.
Figure 3. Total number of CD4+ and CD8+ IEL following treatment with anti-α^{E}β₇ mAb. IEL were isolated, counted and analyzed for CD4+ and CD8+ phenotype by flow cytometry (see Examples). Total no. of IEL was analyzed from IL-2-/- mice that were injected with PBS + Rat IgG2a (n=3; white bars), TNP-OVA in CFA + Rat IgG2a (n=4; black bars) and TNP-OVA in CFA + anti-α^{E}β₇ (n=4; dotted bars).

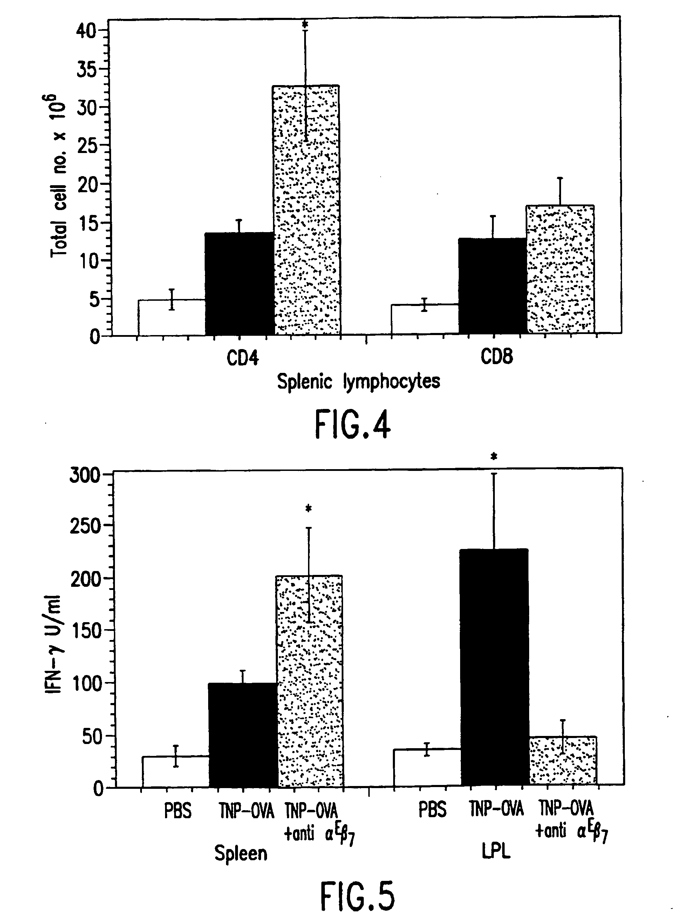

Figure 4. Increased number of CD4+ lymphocytes within the spleen following treatment with α^{E}β₇ mAb. Splenic lymphocytes were isolated, counted and analyzed for CD4+ and CD8+ phenotype by flow cytometry (see Examples). Total no. of splenic lymphocytes was analyzed from IL-2-/- mice that were injected with PBS + Rat IgG2a (n=3; white bars), TNP-OVA in CFA + Rat IgG2a (n=4; black bars) and TNP-OVA in CFA + anti-α^{E}β₇ (n=4; dotted bars). *p < 0.01.
Figure 5. Increased IFN-γ production of splenic lymphocytes following treatment with anti-α^{E}β7 mAb. Splenic lymphocytes were isolated from IL-2-/- mice that were injected with PBS + Rat IgG2a (n=3; white bars), TNP-OVA in CFA + Rat IgG2a (n=4; black bars) and TNP-OVA in CFA + anti-α^{E}β₇ (n=4; dotted bars). Splenic lymphocytes were then stimulated in vitro with anti-CD3 + anti-CD28 and IFN-γ production was analyzed by ELISA in cell culture supernatants after 48 hours (see Examples). *p < 0.01.

### DESCRIPTION OF THE PREFERRED EMBODIMENTS

The present invention may be understood more readily by reference to the following detailed description of the preferred embodiments of the invention and the Example included therein.

Before the present compounds and methods are disclosed and described, it is to be understood that this invention is not limited to specific proteins or specific methods. It is also to be understood that the terminology used herein is for the purpose of describing particular embodiments only and is not intended to be limiting.

It must be noted that, as used in the specification and the appended claims, the singular forms "a," "an," and "the" include plural referents unless the context clearly dictates otherwise.

The present invention provides the use of an antibody to α^{E}β₇ as defined in claim 1.

In the present invention, the subject can be be any mammal, preferably human, and can include but is not limited to mouse, rat, guinea pig, hamster, rabbit, cat, dog, goat, monkey, horse and chimpanzee.

As used herein, "treating" describes an improvement in the patient's clinical state. The improvement may range from reduction of the inflammatory response to complete amelioration of the inflammatory disesase.

As used herein, "autoimmune disease" describes a disease state or syndrome whereby a subject's body produces a dysfunctional immune response against the subject's own body components, with adverse effects. This may include production of B cells which produce antibodies with specificity for all antigens, allergens or major histocompatibility (MHC) antigens, or it may include production of T cells bearing receptors that recognize self-components and produce cytokines that cause inflammation. Examples of autoimmune diseases include, but are not limited to, ulcerative colitis, Crohn's disease, multiple sclerosis, rheumatoid arthritis, diabetes mellitus, pernicious anemia, autoimmune gastritis, psoriasis, Bechet's disease, Wegener's granulomatosis, Sarcoidois, autoimmune thyroiditis, autoimmune oophoritis, bullous pemphigoid, phemphigus, polyendocrinopathies, Still's disease, Lambert-Eaton myasthenia syndrome, myasthenia gravis, Goodpasture's syndrome, autoimmune orchitis, autoimmune uveitis, systemic lupus erythematosus, Sjogren's Syndrome and ankylosing spondylitis (15-22).

As used herein, "allergic disease" describes a disease state or syndrome whereby the body produces a dysfunctional immune response composed of Immunoglobulin E (IgE) antibodies to environmental antigens and which evoke allergic symptoms. Examples of allergic disease include, but are not limited to, asthma, ragweed pollen hayfever, allergy to food substances, atopic eczema, hypersensitivity pneumonitis, Farmers lung, Hyper eosinophilic syndromes and allergic reactions (23-25).

As used herein, "graft-versus-host" (GvH) disease describes a disease state or syndrome whereby an immune response is initiated by grafted cells and is directed against the subject's body with adverse effects. Examples of GvH disease include, but are not limited to, acute and chronic GvH disease following bone marrow transplant (26).

Transplantation rejection describes a disease state or syndrome whereby the transplant recipient's body produces an immune response against the engrafted tissue, resulting in rejection. Transplantation rejection can occur, for example, with kidney, heart, lung or liver transplants as well as with any other transplanted tissue (27,32).

The antagonist of α^{E}β₇ can be an antibody, either polyclonal or monoclonal, that specifically binds with α^{E}β₇ or a ligand that binds to α^{E}β₇. Anti-idiotypic antibodies and affinity matured antibodies are also considered. Other antagonists of α^{E}β₇ can include, but are not limited to inhibitors of α^{E}β₇ production, inhibitors of α^{E}β₇, expression, and molecules designed to block α^{E}β₇ activation or binding. The antagonist can be a whole protein or a fragment of a protein that inhibits the activity, production, expression, activation and binding of α^{E}β₇. For example, a molecule that blocks α^{E}β₇ binding can be a molecule that binds to the cellular receptor for α^{E}β₇. Crystal structures of α^{E}β₇ and its ligand, E-cadherin, may be utilized to design molecules that may disrupt the α^{E}β₇-ligand interaction.

The phrase "specifically binds" with the α^{E}β₇ refers to a binding reaction which is determinative of the presence of the α^{E}β₇, in a heterogeneous population of proteins and other biologics. Thus, under designated conditions, the specified antibodies bound to a particular protein (e.g. α^{E}β₇ or the cellular receptor for α^{E}β₇) do not bind in a significant amount to other proteins present in the subject. Selective binding to an antibody under such conditions may require an antibody that is selected for its specificity for a particular protein. A variety of immunoassay formats may be used to select antibodies that selectively bind with a particular protein (e.g. α^{E}β₇ or the cellular receptor for α^{E}β₇). For example, solid-phase ELISA immunoassays are routinely used to select antibodies selectively immunoreactive with a protein. See *Harlow and Lane* "Antibodies, A Laboratory Manual" Cold Spring Harbor Publications, New York, (1988), for a description of immunoassay formats and conditions that could be used to determine selective binding.

The antibodies of this invention can be from any source. However, to reduce the immunogenicity of the immunoglobulins themselves, antibodies are preferably of human origin or are antibodies generated in other species and "humanized" for administration in humans as described in the Example. The antibodies of this invention can be fragments which retain the ability to bind their specific antigens. For example, fragments of antibodies which maintain α^{E}β₇ binding activity, as well as fragments of α^{E}β₇ which maintain α^{E}β₇ binding activity (e.g. homodimer formation) but which function to inhibit the receptor-ligand binding interaction and activity of α^{E}β₇, are included within the meaning of the term "antibody". Such antibodies and fragments can be made by techniques known in the art and screened for specificity and activity according to the methods set forth in the Example herein. For example, general methods for producing antibodies can be found in *Harlow and Lane* "Antibodies, A Laboratory Manual" Cold Spring Harbor Publications, New York, (1988).

In the present invention, the antagonist of α^{E}β₇ can be orally or parenterally administered in a carrier pharmaceutically acceptable to human subjects. The antagonist of α^{E}β₇ can also be administered in an enema, or as an inhalant, nasal spray or eye drops. Suitable carriers for oral or inhaled administration of the antagonist of α^{E}β₇ can include one or more of the carriers pharmaceutically acceptable to human subjects. Suitable carriers for oral administration of the antigen include one or more substances which may also act as a flavoring agents, lubricants, suspending agents, or as protectants. Suitable solid carriers include calcium phosphate, calcium carbonate, magnesium stearate, sugars, starch, gelatin, cellulose, carboxypolymethylene, or cyclodextrans. Suitable liquid carriers may be water, pyrogen free saline, pharmaceutically accepted oils, or a mixture of any of these. The liquid can also contain other suitable pharmaceutical addition such as buffers, preservatives, flavoring agents, viscosity or osmo-regulators, stabilizers or suspending agents. Examples of suitable liquid carriers include water with or without various additives, including carboxypolymethylene as a ph-regulated gel. The antibody may be contained in enteric coated capsules that release the antigen into the intestine to avoid gastric breakdown. For parenteral administration of the antagonist, a sterile solution or suspension is prepared in saline that may contain additives, such as ethyl oleate or isopropyl myristate, and can be injected for example, into subcutaneous or intramuscular tissues, as well as intravenously. The antagonist of α^{E}β₇ or its ligand may be contained in enteric coated capsules that release the inhibitor into the intestine to avoid gastric breakdown.

Alternatively, the antagonist of α^{E}β₇ may be microencapsulated with either a natural or a synthetic polymer into microparticles 4-8 *µ*m in diameter, which target intestinal lymphoid tissues and produce a sustained release of inhibitor for up to four weeks.

In addition to the oral administration of antigen to a human subject, antagonist to α^{E}β₇ or its ligand, in soluble form, would typically be administered parenterally in a single or multiple dosages of between 0.1 mg and 100 mg/kg of body weight, with preferred dosage range of 1-50 mg/kg and most preferred dosage of 2-10 mg/kg. Subjects can be given antibodies to α^{E}β₇ as a single injection approximately weekly for between one and 52 weeks. For oral administration, 500 mg to 1000 mg of antibodies to α^{E}β₇ can be given PO. For administration of antibodies to α^{E}β₇ in particulate form, 500 mg to 1000 mg can be microencapsulated as described for slow release over a four to eight week period. The treatment can be reinitiated at any time, particularly in the event of a recurrence. Depending on the particular inflammatory disease and the patient's condition, a maintenance dosage may be administered to prevent reoccurence. This dosage can be administered every other week to monthly depending on the nature of the inflammation which may vary from an acute condition to a maintenance state.

The amount of antagonist of α^{E}β₇ administered will vary among individuals based on age, size, weight, condition, etc. One skilled in the art will realize that dosages are best optimized by the practicing physician and methods for determining dosage are described, for example in Remington's Pharmaceutical Science.

The efficacy of administration of a particular dose of an antagonist of α^{E}β₇ in treating an autoimmune disease, allergic disease, GvH disease or transplantation rejection in a subject diagnosed as having an autoimmune disease, allergic disease, GvH disease or transplantation can be determined by standard methods of evaluation of the particular signs, symptoms and objective laboratory tests for a particular disease, as known in the art. For example, if 1) a subject's frequency or severity of recurrences is shown to be improved, 2) the progression of the disease is shown to be stabilized, or 3) the need for use of other immunosuppressive medications is lessened, based on a comparison with an appropriate control group, and knowledge of the normal progression of disease in the general population or the particular individual, then a particular treatment will be considered efficacious.

The efficacy of an antagonist of α^{E}β₇ in preventing an autoimmune disease, allergic disease, GvH disease or transplantation rejection in a subject not known to have an autoimmune disease, allergic disease, GvH disease or transplantation rejection can be determined by evaluating standard signs, symptoms and objective laboratory tests, as would be known to one of skill in the art, over time. This time interval may be large, with respect to the development of an autoimmune disease or allergic disease (years/decades) or short (weeks/months) with respect to the development of GvH disease or transplantation rejection. The determination of who would be at risk for the development of an autoimmune disease or allergic disease would be made based on current knowledge of the known risk factors for a particular disease familiar to a clinician in this field, such as a particularly strong family history of disease. For GvH disease and transplantation rejection, patients undergoing transplant procedures would be considered at risk for the development of these diseases.

The invention provides the use of αEβ7 for preparing a medicament wherein the medicament comprises another therapeutic agent. The invention also provides the use as above for preventing inflammation.

Other therapeutic agents may include, but are not limited to, antibodies, such as anti-α⁴β₇ Mab, cytokines, immunosuppressive agents or immunomodulatory agents.

Examples of immunosuppressive medication include, but are not limited to, corticosteroids, Mofetil, cytotoxic drugs (Azathioprine, Chlorambucil, Cyclophosphamide, Methotrexate, Cyclosporin, FK-506, 6-MP), and Thalidomide (27-28). An example of combination therapy with corticosteroids would include the administration of an inhibitor to α^{E}β₇ or its ligand with the combination of corticosteroids in either a low dose 2.5-20 mg/daily or a high dose 0.5-2 mg/Kg/day regimen, orally or intravenously. This would also include the use of corticosteroids in any other carrier such as an inhalants or an enema. An example of combination therapy with azathioprine would be an oral administration of 1.25-2.5 mg/Kg/day. An example of combination therapy with cyclophosphamide would be an oral dose of 1-3 mg/kg/day or an intravenous dose of 10 -20 mg/Kg/every 1-3 months. An example of combination therapy with Methotrexate would be an oral regimen of 2.5 - 25 mg weekly.

Examples of immunomodulatory agents include, but are not limited to, IVIG, administration of antisera against lymphocyte membrane antigens (i.e. antithymocyte serum (ATS), antithymocyte globulin (ATG), antilymphocyte serum (ALS), antilymphocyte globulin (ALG), anti-CD3, anti-CD4, anti-CD8)), anti-TNFα, anti-IL-12, anti-IFNγ, antisense STAT4 oligonucleotides, anti-ICAM1, antisense ICAM-1 oligonucleotides, anti-CD40L, anti-CD25 (anti-Tag), and IL-10 (29-30). Immunomodulators can be administered both to treat an acute episode of disease or to maintain the patient.

The efficacy of administration of a combination therapy as described herein in treating an autoimmune disease, allergic disease, GvH disease or transplantation rejection in a subject diagnosed as having an autoimmune disease, allergic disease, GvH disease or transplantation can be determined by standard methods of evaluation of the particular signs, symptoms and objective laboratory tests for a particular disease, as known in the art. For example, 1) a subject's frequency or severity of recurrences is shown to be improved, 2) the progression of the disease is shown to be stabilized, or 3) the need for use of other immunosuppressive medications is lessened, based on a comparison with an appropriate control group and knowledge of the normal progression of disease in the general population or the particular individual, then a particular treatment will be considered efficacious.

The efficacy of combination therapy with any antibodies, immunosuppressive medications or immunomodulatory agents and an antagonist of α^{E}β₇ in preventing an autoimmune disease, allergic disease, GvH disease or transplantation rejection in a subject not known to have an autoimmune disease, allergic disease, GvH disease or transplantation rejection can be determined by evaluating standard signs, symptoms and objective laboratory tests, as would be known to one of skill in the art, over time. This time interval may be large, with respect to the development of an autoimmune disease or allergic disease (years/decades) or short (weeks/months) with respect to the development of GvH disease or transplantation rejection. The determination of who would be at risk for the development of an autoimmune disease or allergic disease would be made based on current knowledge of the known risk factors for a particular disease familiar to a clinician in this field, such as a particularly strong family history of disease. For GvH disease and transplantation rejection, patients undergoing transplant procedures would be considered at risk for the development of these diseases.

According to the present invention compositions comprising an α^{E}β₇ antibody and a pharmaceutically acceptable carrier are used. By "pharmaceutically acceptable" is meant a material that is not biologically or otherwise undesirable, i.e., the material may be administered to a subject, along with the antagonist without causing any undesirable biological effects or interacting in a deleterious manner with any of the other components of the pharmaceutical composition in which it is contained. The carrier would naturally be selected to minimize any degradation of the active ingredient and to minimize any adverse side effects in the subject, as would be recognized by one skilled in the art.

Also used are compositions comprising an αEβ7 antibody and a pharmaceutically acceptable carrier, wherein the carrier is not hybridoma supernatant. In this invention, a pharmaceutically acceptable carrier would not be ascites fluid.

In another embodiment, the present invention uses an α^{E}β₇ antibody, a second anti-inflammatory agent and a pharmaceutically acceptable carrier as defined in claim 8. After having administered

In another embodiment, the present invention provides a method of screening for a substance effective in reducing the inflammatory effects of α^{E}β₇, the substance to an animal having an established inflammatory disease; inflammatory tissue cells from the animal are assayed for an amount of secretion of proinflammatory cytokines, inflammatory cytokines or chemokines, whereby a decrease in the amount of proinflammatory cytokines, inflammatory cytokines or chemokines secreted by the inflammatory tissue cells of the animal as compared to the amount of proinflammatory cytokines, inflammatory cytokines or chemokines secreted by inflammatory tissue cells of a control animal having an inflammatory disease and without having the substance administered indicates the substance is effective in reducing the inflammatory effect of αEβ7. The method further comprises the step of evaluating the effect of the antagonist of αEβ7 in reducing the adhesion/retention/influx of αEβ7+ inflammatory cells into the inflammatory tissue. A substance effective in reducing the inflammatory response of an established inflammatory disease is one that reduces or reverses the histological and clinical manifestations of the inflammatory disease, as would be recognized by someone skilled in the art. Furthermore, an independent method comprising determining the effect of a substance on the adhesion, retention, or influx of αEβ7 inflammatory cells can be used to identify antagonists of αEβ7.

In assaying inflammatory tissue cells, several inflammatory cytokines and chemokines can be assayed. These include, but are not limited to IFN-γ, TNF-α, II-4, IL-5. or IL-12, depending on the inflammatory disease in question.

Also provided by the present invention is a method of screening for a substance effective in preventing the inflammatory effects of α^{E}β₇, as defined in claim 12. After having administered the substance to an animal susceptible to an inflammatory disease; the animal is subjected to treatment that will induce an inflammatory response; inflammatory tissue cells from the animal are assayed for an amount of secretion of proinflammatory cytokines, inflammatory cytokines or chemokines, whereby a lack of increase in the amount of proinflammatory cytokines, inflammatory cytokines or chemokines secreted by the inflammatory tissue cells of the animal as compared to an increase in the amount of proinflammatory cytokines, inflammatory cytokines or chemokines secreted by inflammatory tissue cells of a control animal having an inflammatory disease and without having the substance administered indicates the substance is effective in preventing the inflammatory disease by inhibiting the inflammatory effect of α^{E}β₇. The method further comprises the step of evaluating the effect of the antagonist of αEβ7 in reducing the adhesion/retention/influx of αEβ7+ inflammatory cells into the inflammatory tissue. Furthermore, an independent method comprising determining the effect of a substance on the adhesion, retention, or influx of αEβ7 inflammatory cells can be used to identify antagonists of αEβ7.

In the present screening methods, the animal in which the inflammation is produced can be any mammal and can include but is not limited to mouse, rat, guinea pig, hamster, rabbit, cat, dog, goat, monkey, and chimpanzee. The inflammation can be produced in the animal by any method known in the art. For example, the inflammation can be produced by introducing into the animal an effective amount of a hapten reagent. The hapten reagent can be, but is not limited to a 2,4,6-trinitrophenol conjugate of keyhole limpet hemocyanin, 2,4,6-trinitrobenzene sulfonic acid, 2.4,6 dinitrochlorobenzene and other trinitrophenylamine compounds.

Also provided are in vitro screening methods for antagonists to α^{E}β₇. In vitro cellular adhesion studies may be used to analyze the effect of chemically engineered compounds to block the binding of α^{E}β₇ to its ligands or to analyze its ability to reduce cellular activation and pro-and inflammatory cytokine production (29,31,33). Examples of markers for cellular activation include, but.are not limited to, CD25, CD45, CD69 and L-selectin.

Also provided are methods for assaying the amount of pro and inflammatory cytokines, and chemokine secretion from inflammatory tissue obtained from lamina propia, lymph nodes, spleen, liver, skin, lungs, joints, CNS and other tissues involved in inflammatory. These methods include, but are not limited to, flow cytometry, ELISA, PCR, reverse-transcriptase-polymerase chain reaction and ELISPOT, Northern blots, Southern blots, and Western blots (31). A reduced amount of cytokine or chemokine indicates the presence of a substance that may be acting at the level of α^{E}β₇.

The present invention is more particularly described in the following examples which are intended as illustrative only since numerous modifications and variations therein will be apparent to those skilled in the art.

### Examples

### Mice

Mice rendered IL-2 deficient via gene targeting (34) were gaciously donated by W. Paul (NIAID, NIH). Colonies of mice were generated by breeding heterozygous parents, and 4 wk after birth, homozygous mice were identified by PCR analysis of DNA isolated from digested tail snips as described (12). Mice were housed in a specific pathogen free (SPF) animal facility on the Bethesda campus of the National Institutes of Health. Prior to study, all mice were handled with gloves under a class II hood, fed sterile food and water ad libitum, and maintained in sterile microisolators that were changed weekly. Selected tissues of various mice were collected for histopathology and endotape testing; cecum and colons were collected for parasitology. Such tissues tested negative for mouse adenoviruses, carbocillus, ectromelia, EDIM (epizootic diarrhea of infant mice), GDVII (George disease, VII), lymphocytomegalovirus, mouse hepatitis virus, *Mycoplasma,* mouse pneumonia virus, reovirus, sendavirus, mouse cytomegalovirus, and parvovirus. Mice of both sexes aged 4-8 wk were used.

### Production of humanized mouse antibodies to α^{E}β₇.

Rodent monoclonal or polyclonal antibodies can be modified according to the protocols set forth in Junghans et.al. (36), Brown et.al. (37), Kettleborough et.al. (38). Specifically, rodent antibodies can be modified for human administration by constructing, through recombinant DNA protocols known to one of skill in the art, a chimeric rodent-human antibody composed of rodent variable regions and human heavy and light chain constant regions. Another approach to humanizing rodent antibodies is to graft rodent complementary-determining regions (CDRs) from the rodent variable regions into human variable regions. By using either of these approaches, rodent antibodies can be humanized for administration into human subjects.

### Immunization and treatment of mice

Mice (4-8 weeks) were immunized once with 2,4,6-trinitrophenol (TNP) conjugate of keyhole limpet hemocyanin (KLH) in complete Freund's adjuvant. The conjugate was prepared as follows: 20 mg of OVA (Pierce Chemical Co., Rockford, IL) reconstituted in 2.0 ml distilled water was added to 2.0 ml of potassium borate buffer (pH 9.0-9.3), then mixed with 3.0 ml of 1 mg/ml solution of TNBS (gift from Dr. J. Inman, NIAID, NIH) in distilled water and 8 *µ*l of 1 M Na₂CO₃ added; the mixture was allowed to sit in the dark for 8 h at room temperature and dialyzed 12 h in 1X PBS, after which the concentration of the conjugate was determined via BCA protein assay reagent (Pierce Chemical Co.). Mice were injected i.p. with 100 *µ*g TNP-OVA emulsified in Freund's complete adjuvant (Sigma ImmunoChemicals, St. Louis, MO). For control, mice were injected with PBS emulsified in Freund's complete adjuvant.

To determine the role of α₄β₇ and α^{E}β₇ in the development of colitis in IL-2^{-/-} mice, the latter were administered 0.5 mg of anti-α₄β₇ (Rat IgG2a mAb, Clone DATK32, Pharmingen), anti-α^{E}β₇ (Rat IgG2a mAb, Clone M290, Pharmingen) mAb or Rat-IgG2a κ control Ab (Pharmingen) i.p. 1 h prior to TNP-OVA immunization (day 0) and the same amount of mAbs were readministered on day 2, 4 and 6 until mice were sacrificed. In some studies, groups of mice that had previously been immunized with TNP-OVA and had developed colitis were injected with 0.5 mg of anti-α^{E}β₇ every other day times 3 and evaluated on the 7th day from the first anti-α^{E}β₇ mAb injection. FACS analysis of double-stained of LP cells with anti-α₄β₇, and anti-α^{E}β₇ mAb revealed no cross-reactivity between the anti-α₄β₇ and anti-α^{E}β₇.

### Grading of histology changes

Colons were dissected from immunized mice and processed for the preparation of frozen sections. Briefly, small sections, approximately 3-5 mm, were cut from each tissue and submerged in dry ice-cooled O.C.T. Embedding Compound (Tissue Tek; Miles Inc., Elkhart, IN). After complete solidification of the embedding media, samples were stored at -80°C.

Tissues were removed at indicated time points, cryopreserved, fixed, and embedded in paraffin. The embedded tissues were sectioned and stained with hematoxylin and eosin (American Histo Labs, Rockville, MD). The degree of inflammation of the colon was graded semiquantitatively from 0 to 4 (0, no signs of inflammation; 1, very low level and 2, low level of mononuclear cell infiltration; 3, high level of mononuclear cell infiltration, high vascular density, thickening of the colon wall; 4, transmural infiltration, loss of goblet cells, high vascular density, thickening of the colon wall).

### IEL and Lamina propria cell isolation

Preparation of IEL and LP cell has been previously described (12). Briefly, detritus was squeezed out of the colon lumen with forceps, the tissue minced with sterile scissors, and the pieces washed with HBSS over a 100 mm nylon mesh bowl to remove remaining detritus and metabolites. IEL were liberated by incubating the cut pieces in 25 ml of warm (37°C) HBSS media without Ca/Mg (Biofluids, Inc., Rockville, MD) supplemented with 25 mM HEPES buffer (NIH Media Unit) and 10% FCS (Bio Whittaker, Walkersville, MD) containing 0.1M EDTA for 20 min at 37°C. Filtered cells were enriched for lymphocytes by Percoll-gradient centrifugation. For Colonic lamina propria lymphocytes (LPL) isolation, the remaining pieces of colon were washed over nylon mesh, resuspended in RPMI 1640 media (BioWhittaker) augmented with 25 mM HEPES buffer, 10% FCS, 400 U/ml DNAse (Boehringer Mannheim Biochemical, Indianapolis, IN), 400 U/ml collagenase (Boehringer Mannheim), and incubated 1.5 h at 37°C on a rocker. The resultant cell suspension was filtered sequentially through 100 mm and 40 mm nylon mesh filters and then washed 2' in RPMI 1640 supplemented with 25 mM HEPES and 10% FCS.

### In vitro cell stimulation and cytokine assay

Isolated cells were stimulated in RPMI 1640 supplemented with 10% FCS, 5% NCTC (M. A. Whittaker Bioproducts), 1% penicillin/streptomycin/0.25% fungisozone (GibcoBRL, Gaithersburg, MD), 2 mM glutamine (GibcoBRL), 5 x 10⁵ M ME (Sigma Chemical Co., St. Louis, MO), and 15 mM HEPES at a density of 1 x 10⁶cells per 1.0 ml final volume. Cells were cultured in 24-well tissue culture plates (Costar, Cambridge, MA) in the presence of plate-bound anti-CD3 (10 *µ*g/ml; Pharmingen) with soluble anti-CD28 (1 *µ*g/ml; Pharmingen) and IL-2 (50 U/ml; Chiron, San Diego, CA) at 37°C in humidified 6% CO₂. Culture supernatants were harvested after 48 h and frozen at -20°C until assayed. IFN-γ production in culture supernatants was assayed by ELISA using the Pharmingen protocol in association with coating, standard, and biotinylated secondary Abs obtained from Pharmingen and HRP-streptavidin obtained from Zymed (South San Francisco, CA).

### Flow cytometry

To characterize T cell phenotypes in recovered LPL, IEL and splenocytes, flow cytometric analysis with Abs to murine cell surface determinants (Pharmingen) was employed. For this purpose, cells were washed 2x in FACS buffer (NIH Media Unit), resuspended at 10⁷ cells/ml in FACS buffer, and transferred to FACS tubes (Becton Dickinson, Mountain View, CA). To prevent nonspecific FcR-mediated binding of Abs, 50-100 *µ*g/ml of anti-FcεR (2.4G2; Pharmingen) was added to each tube 3 min prior to staining. Cell suspensions were stained with 1 *µ*g/ml for 30 min on ice with FITC-conjugated CD3, FITC-conjugated CD4, PE-conjugated CD8, PE-conjugated α₄β₇, biotin-conjugated ICAM-1 (CD54), or biotin-conjugated CD69. Cells stained with biotin-conjugated Abs were subsequently washed once in FACS buffer and incubated on ice for 30 min with streptavidin-PE at 5 *µ*g/ml. All stained cells were washed 2x in FACS buffer, resuspended at 1 x 10⁶ cells/ml, and examined on a FACSCAN analyzer, Lysis II software (Becton Dickinson). Nonviable cells were excluded by forward angle scatter or by propidium iodide uptake.

### Statistical analysis

Descriptive statistics and testing for significance of differences were assessed by Student's *t* test using the StatWorks and Microsoft Excel statistical analysis computer programs.

### Treatment of TNP-OVA induced colitis by either anti- α^{E}β₇ or anti-α₄β₇ mAb is associated with reduction of LPL and IEL cell numbers, but increase of splenic cell numbers.

To evaluate the role of α^{E}β₇ vs. α₄β₇ in a newly developed TNP-OVA/IL-2-/- model of colitis, IL-2-/- mice maintained under specific pathogen-free condition were immunized with TNP-OVA and given either anti-α^{E}β₇ or anti-α₄β₇ mAbs at the same time of immunization, as described above. Therefore, colitis was induced in IL-2-/- mice that were maintained in specific pathogen-free environment and evaluated the affect of concomitant injection of α^{E}β₇ mAb. As shown in Figure 1, severe colitis was seen in mice injected with TNP-OVA, however, when TNP-OVA was co-injected with anti-α^{E}β₇ mAb the colitis was prevented and microscopic examination of the colonic mucosa resembled the one seen in PBS-injected mice. This was reflected by significant reduction in colitis severity score (CSS); IL-2-/- mice injected with TNP-OVA, CSS: 3-4 vs. TNP-OVA + anti-α^{E}β₇ injected, CSS: 0-2 (Tablel). Furthermore, this inhibition of colitis was associated with a fourfold reduction in LPL numbers and twofold reduction in IEL numbers in mice that were co-injected with α^{E}β₇ mAb (IL-2-/- LPL: TNP-OVA; 180±5 x 10⁶ vs. TNP-OVA co-injected with anti-α^{E}β₇ 6.5±0.6 x 10⁶. IL-2-/- IEL: TNP-OVA; 5.2±1.8 x 10⁶ vs. TNP-OVA co-injected with anti-α^{E}β₇; 2.4±0.9 x 10⁶). Interestingly, when the total number of cells were looked at within the spleen there was a slight increase of total cell numbers (Tablel). Similarly, TNP-OVA-induced colitis was significantly reduced when mice with established colitis were treated with two anti-α^{E}β₇ mAb injections, TNP-OVA CSS: 3-4 vs. TNP-OVA + anti-α^{E}β₇ mAb injections CSS: 1-2. To evaluate the role of α₄β₇ in this model of colitis, IL-2-/- mice were co-injected with TNP-OVA and anti-α₄β₇. As shown in Table 1, injection of anti-α^{E}β₇ also prevented the development of TNP-OVA-induced colitis in IL-2-/- mice. Therefore, the above data demonstrates that not only is the injection of anti-α^{E}β₇ mAb capable of preventing TNP-OVA-induced colitis in IL-2-/- mice, but can also directly ameliorate established colitis.

### Administration of anti-α^{E}β₇ mAb in to IL-2-/- mice reduces the number of infiltrating CD4+ T cells within the LPL, but increases CD4+ T cells within the spleen.

It has previously been demonstrated that CD4+ T cells are the principal effector cells involved in the pathogenesis of TNP-OVA-induced colitis in IL-2-/- mice(12, 17). Therefore it is interesting to see if the administration of anti-α^{E}β₇ vs. anti-α₄β₇ had different effects on lymphoid organ compartmentalization of CD4+ and CD8+ T cells. As shown in Table 1, injection of anti-α^{E}β₇ mAb was most associated with reduction of total number of cells within the LP compartment. This reflects previous observations (and demonstrated here in Table 1 and figure 1) that the most severe inflammation is found within the LP of the colon. Furthermore, as shown in figure 2, anti-α^{E}β₇ mAb injection led to a five-fold reduction of LP CD4+ lymphocytes; total number of CD4+ LPL in TNP-OVA vs. TNP-OVA + anti-α^{E}β₇, 21.7 x 10⁶ and 4.7 x 10⁶, respectively. Within the IEL compartment anti-IEL treatment was associated with modest reduction of CD8+ T cells; TNP-OVA: 4 x 10⁶ vs. TNP-OVA + anti-α^{E}β₇: 1.6 x 10⁶ (figure 3). Interestingly, the total number of CD4+ T cells within the IE compartment was not significantly altered by the injection of anti-α^{E}β₇ mAb into IL-2-/- mice. Furthermore, when we analyzed similarly treated IL-2+/+ mice no changes in the total number of CD4+ T cells were observed within any of the compartments evaluated (Table 1).

To further evaluate if the reduction of CD4+ lymphocytes in the LP of TNP-OVA immunized IL-2-/- mice following anti-α^{E}β₇ mAb treatment was due to the deletion of α^{E}β₇ + lymphocytes or rather an effect on lymphocyte distribution, the number of T cells within the peripheral lymphoid organs was also evaluated. As shown in figure 4, injection of anti-α^{E}β₇ was associated with significantly increased numbers of CD4+ T cells within the spleen; TNP-OVA: 13.5 x 10⁶ vs. TNP-OVA + anti-α^{E}β₇: 32.4 x 10⁶. Within the mesenteric lymph node compartment total CD4+ T cell numbers were similar in TNP-OVA immunized IL-2-/- mice whether or not the had received anti-α^{E}β₇ or not.

### Administration of α^{E}β₇ mAb results in decreased IFN-γ production within LPL but increased IFN-γ production within the Spleen

Since it is now well established that the severity of chronic mucosal inflammation correlates with the level of IFN-γ production by inflammatory CD4+ T cells in many murine models of colitis whether the treatment with anti-α^{E}β₇ mAb affects IFN-γ levels was further analyzed. Thus, in initial studies the IFN-γ production of LPL isolated from treated mice were compared with LPL isolated from untreated mice. After isolation, LPL were directly stimulated *in vitro* with anti-CD3 and anti-CD28 mAbs and IFN-γ production in the culture supernatants was analyzed. As demonstrated in figure 5, T cells from anti-α^{E}β₇ mAb treated IL-2-/- mice manifested a five-fold reduction of IFN-γ when compared to untreated TNP-OVA immunized mice: TNP-OVA + anti-α^{E}β₇, mAb: IFN-γ 46±15 U/ml vs. IFN-γ 225±72 U/ml (p = 0.003). In a similar series of studies, splenic T cell IFN-γ production from treated vs. untreated IL-2-/- mice was evaluated. As shown in figure 5, TNP-OVA immunization alone induced a three-fold increase in IFN-γ production in the splenic T cells when compared to PBS injected control IL-2-/- mice. Furthermore, this amount of splenic IFN-γ secretion was even higher, following co-injection of TNP-OVA and anti-α^{E}β₇; TNP-OVA alone: IFN-γ 97±20 U/ml vs. TNP-OVA + anti-α^{E}β₇ mAb: IFN-γ 202±14 U/ml, p = 0.005. Of note, in none of the above conditions could we find a significant IL-4 production.

Above data indicate that administration of anti-α^{E}β₇ mAb is preventing the influx and/or retention of T cells that are committed to the Th1 cytokine pathway into the colonic LP.

### Anti-α^{E}β₇ mAb treatment prevents accumulation of GD3+/CD54+ LPL in TNP-OVA immunized IL-2-/- mice.

The expression of adhesion molecules is undoubtedly an important regulatory element during the induction of chronic mucosal inflammation. ICAM-1 (CD54) is one member of the Immunoglobulin superfamily that is significantly up-regulated on T cells during Th1-mediated inflammation and the binding to its ligand VLA-1 has been demonstrated not only to enhance T cell adhesion but also to induce intracellular T cell activation (27-32). Furthermore, it has previously been shown that one of the characteristics of the TNP-OVA-induced inflammatory response in IL-2-/- mice is the induction of CD54 and CD69(21, 26). Since anti-α^{E}β₇ mAb treatment lead only to decreased IFN-γ production within the LP compartment but not in the spleen we were interested to see if this was reflected by the expression of the above described T cell activation markers. As demonstrated in Table 2, there was a significant decrease of CD3+/CD54+ LPL following co-injection of TNP-OVA and anti-α^{E}β₇ compared to TNP-OVA alone, 16.4 x 10⁶ and 2.6 x 10⁶, respectively, and this was also evident by fewer number of LPL expressing CD54 following anti-α^{E}β₇ treatment. However, when the T cell expression of CD54 within the spleen was evaluated there was a notable increase of CD3+/CD54+ lymphocytes following anti-α^{E}β₇ treatment; total number of CD3+/CD54+ lymphocytes following co-injection of TNP-OVA and anti-α^{E}β₇ mAb 165.6 x 10⁶ vs. TNP-OVA alone 135 x 10⁶. Similar findings were seen for the expression of another, but less characterized, activation marker, CD69 (Table 2).

Although the present process has been described with reference to specific details of certain embodiments thereof, it is not intended that such details should be regarded as limitations upon the scope of the invention except as and to the extent that they are included in the accompanying claims.

**Table 1. α^{E}β₇ prevents TNP-OVA induced colitis in IL-2-/- mice**

| | | Spleen Cell no. x10⁶ | LPL Cell no. x10⁶ | IEL Cell no. x10⁶ | Colitis Severity Score | n |
|---|---|---|---|---|---|---|
| IL-2-/- | PBS | 40±0.8 | 3.6±2.1 | 1.7±1.1 | 0-1 | 3 |
| | TNP-OVA | 150±4 | 24.9±10 | 5.2±1.8 | 3-4 | 4 |
| | TNP-OVA + | | | | | 4 |
| | anti-α^{E}β₇ | 180±5 | 6.5±2.6 | 2.4±0.9 | 0-2 | |
| | TNP-OVA + | | | | | 3 |
| | anti-α₄β₇ | 178±9 | 7.1±3.5 | 2.0±1.1 | 0-2 | |
| IL-2+/+ | PBS | 6.8±0.6 | 3.1±0.5 | 2.2±0.9 | 0 | 4 |
| | TNP-OVA | 8.2±1.1 | 3.5±0.8 | 1.9±1.2 | 0 | 4 |

**Table 2. T cells activation following anti-α^{E}β₇ administration**

| | | Spleen CD3+ | | LPL CD3+ | | IEL CD3+ | |
|---|---|---|---|---|---|---|---|
| | % Positive | CD54 | CD69 | CD54 | CD69 | CD54 | CD69 |
| IL-2-/- | PBS | 38±6 | 35±3 | 25±5 | 29±5 | 6 | n.d. |
| | TNP-OVA | 90±3 | 70±1 | 66±7 | 71±5 | 74±8 | n.d. |
| | TNP-OVA + anti-α^{E}β₇ | 92±4 | 66±4 | 40±5 | 60±2 | 64±9 | n.d. |
| IL-2+/+ | PBS | 28±4 | n.d. | 20±3 | n.d. | 33±6 | n.d. |
| | TNP-OVA | 31±6 | n.d. | 28±4 | n.d. | 35±3 | n.d. |

### References

1. Fiocchi, C. 1997. The immune system in inflammatory bowel disease. *Acta Gastroenterol Belg* 60:156.
2. Ludviksson, B.R., R.O. Erhardt, I.J. Fuss, and W. Strober. 1997. Mucosal and Thymic Dysregulation-Role in Human Intestinal Inflammation. *The Immunologist* 5:202.
3. Pedersen, G., J. Brynskov, O.H. Nielsen, and K. Bendtzen. 1995. Adhesion molecules in inflammatory and neoplastic intestinal diseases. *Dig Dis* 13:322.
4. Zeitz, M., G. Kohne, T. Schneider, and M. Zeitz. 1997. Pathogenesis of inflammatory bowel disease. Special features of the intestinal lymphocytic system. *Digestion* 58 Suppl 1:59.
5. Abitorabi, M.A., C.R. Mackay, E.H. Jerome, O. Osorio, E.C. Butcher, and D.J. Erle. 1996. Differential expression of homing molecules on recirculating lymphocytes from sheep gut, peripheral, and lung lymph. *J Immunol* 156:3111.
6. Berlin, C., E.L. Berg, M.J. Briskin, D.P. Andrew, P.J. Kilshaw, B. Holzmann, I.L. Weissman, A. Hamann, and E.C. Butcher. 1993. Alpha 4 beta 7 integrin mediates lymphocyte binding to the mucosal vascular addressin MAdCAM-1. *Cell* 74:185.
7. Berlin, C., R.F. Bargatze, J.J. Campbell, U.H. von Andrian, M.C. Szabo, S.R. Hasslen, R.D. Nelson, E.L. Berg, S.L. Erlandsen, and E.C. Butcher. 1995. alpha 4 integrins mediate lymphocyte attachment and rolling under physiologic flow. *Cell* 80:413.
8. Briskin, M., D. Winsor-Hines, A. Shyjan, N. Cochran, S. Bloom, J. Wilson, L.M. McEvoy, E.C. Butcher, N. Kassam, C.R. Mackay, W. Newman, and D.J. Ringler. 1997. Human mucosal addressin cell adhesion molecule-1 is preferentially expressed in intestinal tract and associated lymphoid tissue. *Am J Pathol* 151:97.
9. Parker, C.M., K.L. Cepek, G.J. Russell, S.K. Shaw, D.N. Posnett, R. Schwarting, and M.B. Brenner. 1992. A family of beta 7 integrins on human mucosal lymphocytes. *Proc Natl Acad Sci U S A* 89:1924.
10. Mebius, R.E., P.R. Streeter, S. Michie, E.C. Butcher, and LL. Weissman. 1996. A developmental switch in lymphocyte homing receptor and endothelial vascular addressin expression regulates lymphocyte homing and permits CD4+ CD3- cells to colonize lymph nodes. *Proc Natl Acad Sci U S A* 93:11019.
11. Shaw, S.K., and M.B. Brenner. 1995. The beta 7 integrins in mucosal homing and retention. *Semin Immunol* 7:335.
12. Ehrhardt, R.O., B.R. Ludviksson, B. Gray, M. Neurath, and W. Strober. 1997. Induction and prevention of colonic inflammation in IL-2-deficient mice. *J Immunol* 158:566.
13. Autenrieth, I.B., N. Bucheler, E. Bohn, G. Heinze, and I. Horak. 1997. Cytokine mRNA expression in intestinal tissue of interleukin-2 deficient mice with bowel inflammation. *Gut* 41:793.
14. Ma, A., M. Datta, E. Margosian, J. Chen, and I. Horak. 1995. T cells, but not B cells, are required for bowel inflammation in interleukin 2-deficient mice. *J Exp Med* 182:1567.
15. Greenberger, P.A. 1997. Immunologic aspects of lung diseases and cystic fibrosis. *Jama* 278:1924-30.
16. King, C., and N. Sarvetnick. 1997. Organ-specific autoimmunity. *Curr Opin Immunol* 9:863-71.
17. Ledford, D. K. 1997. Immunologic aspects of vasculitis and cardiovascular disease. *Jama* 278:1962-71.
18.. O'Garra, A., L. Steinman, and K. Gijbels. 1997. CD4+ T-cell subsets in autoimmunity. *Curr Opin Immunol* 9:872-83.
19. Jorgensen, C., A. Travaglio-Encinoza, C. Bologna, A. D. D'Angeac, T. Reme, and J. Sany. 1994. Human mucosyl lymphocyte marker expression in synovial fluid lymphocytes of patient with rheumatoid arthritis. *J Rheumatol* 21:1602-7.
20. Jorgensen, C., I. Couret, I. Hellier, C. Bologna, F. Canovas, J. Brochier, T. Reme, and J. Sany. 1996. In vivo migration of radiolabelled lymphocytes in rheumatoid synovial tissue engrafted in SCID mice: implication of beta 2 and beta 7- integrin. J *Rheumatol 23:32-5*.
21. Trollmo, C., I. M. Nilsson, C. Sollerman, and A. Tarkowski. 1996. Expression of the mucosal lymphocyte integrin alpha E beta 7 and its ligand E-cadherin in the synovium of patients with rheumatoid arthritis. *Scand J lmmunol* 44:293-8.
22. Ludviksson, B.R., R.O. Ehrhardt, and W. Strober. 1997. TGF-beta production regulates the development of the 2,4,6-trinitrophenol-conjugated keyhole limpet hemocyanin-induced colonic inflammation in IL-2-deficient mice. *J Immunol* 159:3622.
23. Lemanske, R. F., Jr., and W. W. Busse. 1997. Asthma. *Jama 278:1855-73.*
24. Leung, D. Y., L. A. Diaz, V. DeLeo, and N. A. Soter. 1997. Allergic and immunologic skin disorders. *Jama 278:1914-23.*
25. Sampson, H. A. 1997. Food allergy. *Jama 278:1888-94.*
26. Klingebiel, T., and P. G. Schlegel. 1998. GVHD: overview on pathophysiology, incidence, clinical and biological features. *Bone Marrow Transplant 21 Suppl 2: S45-9.*
27. Ballow, M., and R. Nelson. 1997. Immunopharmacology: immunomodulation and immunotherapy [see comments]. *Jama 278:2008-17.*
28. McCune, W. J., and A. W. Friedman. 1992. Immunosuppressive drug therapy for rheumatic disease. *Curr Opin Rheumatol 4:314-21.*
29. Evans, R. C., L. Clarke, P. Heath, S. Stephens, A. I. Morris, and J. M. Rhodes. 1997. Treatment of ulcerative colitis with an engineered human anti-TNFalpha antibody CDP571. *Aliment Pharmacol Ther 11:1031-5*.
30. Yacyshyn, B. R., M. B. Bowen-Yacyshyn, L. Jewell, J. A. Tami, C. F. Bennett, D. L. Kisner, and W. R. Shanahan, Jr. 1998. A placebo-controlled trial of ICAM-1 antisense oligonucleotide in the treatment of Crohn's disease. *Gastroenterology 114:1133-42.*
31. 1994. *Current protocols in Immunology.* Current Protocols.
32. Kahan, B. D., and J. H. Clark III. 1995. Transplantation of solid organs. In *Samter's Immunologic Diseases, vol. II.* M. M. Frank, K. F. Austen, H. N. Claman, and E. R. Unanue, eds. Little, Brown and Company, Boston, p. 1495 - 1509.
33. Cepek, K. L., S. K. Shaw, C. M. Parker, G. J. Russell, J. S. Morrow, D. L. Rimm, and M. B. Brenner. 1994. Adhesion between epithelial cells and T lymphocytes mediated by E- cadherin and the alpha E beta 7 integrin. *Nature 372:190-3.*
34. Schorle, H., T. Holtschke, T. Hunig, A. Schimpl, and I. Horak. 1991. Development and function of T cells in mice rendered interleukin-2 deficient by gene targeting. *Nature* 352, no. 6336:621-4.
35. Martin, E.W. Remington's Pharmaceutical Sciences, Martin, latest edition, Mack Publishing Co., Easton, PA.
36. Junghans et al. 1990. *Cancer Research* 50:1495-1502.
37.. Brown et al. 1991. *Proc*. *Natl*. *Acad*. *Sci*. USA 88:2663-2667.
38. Kettleborough et al. 1991. *Protein Engineering* 4:773-783.

## Claims

1. Use of an αEβ7 antibody for preparing a medicament for treating or preventing inflammatory bowel disease in a subject.

2. The use of claim 1 wherein the antibody is an αEβ7 Mab.

3. The use of claim 1, wherein the medicament further comprises another therapeutic agent.

4. The use of claim 3, wherein the other therapeutic agent is an antibody.

5. The use of claim 4, wherein the antibody is an α4β7 antibody.

6. The use of claim 3, wherein the other therapeutic agent is a cytokine.

7. The use of claim 3, wherein the other therapeutic agent is an immunomodulatory agent.

8. A method of screening for a substance effective in reducing the inflammatory effects of αEβ7 in inflammatory bowel disease, comprising:
a) Assaying inflammatory tissue cells from a non-human animal, which has established inflammatory bowel disease and which has received the substance, for an amount of secretion of proinflammatory cytokines, inflammatory cytokines or chemokines, whereby a decrease in the amount of proinflammatory cytokines, inflammatory cytokines or chemokines secreted by the inflammatory tissue cells of the animal as compared to the amount of proinflammatory cytokines, inflammatory cytokines or chemokines secreted by inflammatory tissue cells of a control animal having an inflammatory bowel disease and without having the substance administered indicates the substance is effective in reducing the inflammatory effect of αEβ7 in inflammatory bowel disease;
b) evaluating the effect of the antagonist of αEβ7 in reducing the adhesion/retention/influx of αEβ7+ inflammatory cells into the inflammatory tissue, a reduction indicating that the antagonist is acting at the level of αEβ7.

9. The method of claim 8 wherein the animal has an established inflammatory disease produced by a hapten reagent.

10. The method of claim 9 wherein the hapten reagent is a 2,4,6 trinitrophenol (TNP) conjugate of keyhole limpet hemocyanin (KLH).

11. The method of claim 10, wherein the animal is a mouse.

12. A method for screening for a substance effective in preventing the inflammatory effects of αEβ7 in inflammatory bowel disease, comprising:
a) Assaying inflammatory tissue cells from a non-human animal which is susceptible to an inflammatory bowel disease and has been subjected to a treatment that induces an inflammatory response, for an amount of secretion of proinflammatory cytokines, inflammatory cytokines or chemokines, whereby a lack of increase in the amount of proinflammatory cytokines, inflammatory cytokines or chemokines secreted by the inflammatory tissue cells of the animal as compared to an increase in the amount of proinflammatory cytokines, inflammatory cytokines or chemokines secreted by inflammatory tissue cells of a control animal having an inflammatory bowel disease and without having the substance administered indicates the substance administered indicates the substance is effective in preventing the inflammatory disease by inhibiting the inflammatory effect of α^{E}β₇ in inflammatory bowel disease;
b) evaluating the effect of the antagonist of αEβ7 in reducing the adhesion/retention/influx of αEβ7+ inflammatory cells into the inflammatory tissue.

13. The method of claim 12, wherein the animal is mouse.

14. The method of claim 13, wherein the treatment that will induce the inflammatory response is the introduction of an effective amount of a hapten reagent.

15. The method of claim 14, wherein the reagent is 2;4,6 trinitrophenol (TNP) conjugate of limpet hemocyanin (KLH).

16. The method of claim 8 wherein the cytokine is IFN-γ.

17. The method of claim 12 wherein the cytokine is IFN-γ.

## Patentansprüche

1. Verwendung eines αEβ7-Antikörpers zur Herstellung eines Arzneimittels zur Behandlung oder Verhütung von entzündlicher Darmkrankheit bei einem Patienten.

2. Verwendung nach Anspruch 1, wobei der Antikörper ein αEβ7-Mab ist.

3. Verwendung nach Anspruch 1, wobei das Arzneimittel außerdem ein weiteres therapeutisches Mittel enthält.

4. Verwendung nach Anspruch 3, wobei das weitere therapeutische Mittel ein Antikörper ist.

5. Verwendung nach Anspruch 4, wobei der Antikörper ein α4β7-Antikörper ist.

6. Verwendung nach Anspruch 3, wobei das weitere therapeutische Mittel ein Cytokin ist.

7. Verwendung nach Anspruch 3, wobei das weitere therapeutische Mittel ein immunmodulatorisches Mittel ist.

8. Verfahren zum Screenen nach einer Substanz, die wirksam ist, um die entzündlichen Wirkungen von αEβ7 bei der entzündlichen Darmkrankheit zu vermindern, das beinhaltet, dass
a) Zellen aus entzündlichem Gewebe von einem nicht-menschlichen Tier, bei dem eine entzündliche Darmkrankheit vorliegt und das die Substanz erhalten hat, auf das Ausmaß an Sekretion von proentzündlichen Cytokinen, entzündlichen Cytokinen oder Chemokinen getestet werden, wobei eine Verringerung der Menge an proentzündlichen Cytokinen, entzündlichen Cytokinen oder Chemokinen, die von den Zellen des entzündlichen Gewebes des Tiers ausgeschieden werden im Vergleich zur Menge an proentzündlichen Cytokinen, entzündlichen Cytokinen oder Chemokinen, die von Zellen des entzündlichen Gewebes eines Kontrolltiers mit einer entzündlichen Darmkrankheit und ohne dass die Substanz verabreicht wurde, ausgeschieden werden, anzeigt, dass die Substanz wirksam ist, um die entzündliche Wirkung von αEβ7 bei der entzündlichen Darmkrankheit zu vermindern;
b) die Wirkung des Antagonisten von αEβ7 bei der Reduktion von Adhäsion/Retention/Influx von αEβ7+ entzündlichen Zellen in das entzündliche Gewebe ausgewertet wird, wobei eine Reduktion anzeigt, dass der Antagonist auf der Ebene von αEβ7 wirkt.

9. Verfahren nach Anspruch 8, wobei das Tier eine etablierte entzündliche Krankheit hat, die durch ein Haptenreagenz erzeugt wird.

10. Verfahren nach Anspruch 9, wobei das Haptenreagenz ein 2,4,6-Trinitrophenol-(TNP)-konjugat von Keyhole-Limpet-Hämocyanin (KLH) ist.

11. Verfahren nach Anspruch 10, wobei das Tier eine Maus ist.

12. Verfahren zum Screenen nach einer Substanz, die wirksam ist, um die entzündlichen Wirkungen von αEβ7 bei entzündlicher Darmkrankheit zu verhüten, das beinhaltet, dass:
a) Zellen aus entzündlichem Gewebe von einem nicht-menschlichen Tier, das für eine entzündliche Darmkrankheit empfänglich ist und einer Behandlung unterzogen wurde, die eine entzündliche Antwort induziert, getestet werden auf das Ausmaß der Sekretion von proentzündlichen Cytokinen, entzündlichen Cytokinen oder Chemokinen, wobei ein mangelnder Anstieg der Menge an proentzündlichen Cytokinen, entzündlichen Cytokinen oder Chemokinen, die von den Zellen des entzündlichen Gewebes des Tiers ausgeschieden werden im Vergleich zu einem Anstieg der Menge von proentzündlichen Cytokinen, entzündlichen Cytokinen oder Chemokinen, die von Zellen des entzündlichen Gewebes eines Kontrolltiers mit einer entzündlichen Darmkrankheit und ohne dass die Substanz verabreicht wurde ausgeschieden werden, anzeigt, dass die verabreichte Substanz wirksam ist, um eine entzündliche Krankheit zu verhüten, indem die entzündliche Wirkung von αEβ7 bei entzündlicher Darmkrankheit gehemmt wird;
b) die Wirkung des Antagonisten von _{α}E_{β}7 bei der Reduktion von Adhäsion/Retention/Influx von αEβ7+ entzündlichen Zellen in das entzündliche Gewebe ausgewertet wird.

13. Verfahren nach Anspruch 12, wobei das Tier eine Maus ist.

14. Verfahren nach Anspruch 13, wobei die Behandlung, die die entzündliche Antwort induziert, das Einführen einer wirksamen Menge eines Haptenreagenzes ist.

15. Verfahren nach Anspruch 14, wobei das Reagenz 2,4,6-Trinitrophenol-(TNP)-konjugat von Limpet-Hämocyanin (KLH) ist.

16. Verfahren nach Anspruch 8, wobei das Cytokin IFN-γ ist.

17. Verfahren nach Anspruch 12, wobei das Cytokin IFN-γ ist.

## Revendications

1. Utilisation d'un anticorps αEβ7 pour préparer un médicament pour traiter ou prévenir une maladie intestinale inflammatoire chez un sujet.

2. Utilisation selon la revendication 1 dans laquelle l'anticorps est un AcM αEβ7.

3. Utilisation selon la revendication 1, dans laquelle le médicament comprend en outre un autre agent thérapeutique.

4. Utilisation selon la revendication 3, dans laquelle l'autre agent thérapeutique est un anticorps.

5. Utilisation selon la revendication 4, dans laquelle l'anticorps est un anticorps α4β7.

6. Utilisation selon la revendication 3, dans laquelle l'autre agent thérapeutique est une cytokine.

7. Utilisation selon la revendication 3, dans laquelle l'autre agent thérapeutique est un agent immunomodulateur.

8. Procédé de criblage à la recherche d'une substance efficace pour réduire les effets inflammatoires d'αEβ7 dans une maladie intestinale inflammatoire, comprenant les étapes consistant à :
a) doser les cellules de tissu inflammatoire d'un animal non humain qui a une maladie intestinale inflammatoire établie et qui a reçu la substance, afin de détecter une quantité de sécrétion de cytokines pro-inflammatoires, de cytokines inflammatoires ou de chimiokines, une diminution de la quantité de cytokines pro-inflammatoires, de cytokines inflammatoires ou de chimiokines sécrétée par les cellules de tissu inflammatoire de l'animal comparativement à la quantité de cytokines pro-inflammatoires, de cytokines inflammatoires ou de chimiokines sécrétée par les cellules de tissu inflammatoire d'un animal de contrôle ayant une maladie intestinale inflammatoire et n'ayant pas reçu la substance indiquant que la substance est efficace pour réduire l'effet inflammatoire d'αEβ7 dans la maladie intestinale inflammatoire ;
b) évaluer l'effet de l'antagoniste d'αEβ7 pour réduire l'adhésion/la rétention/l'influx de cellules inflammatoires αEβ7+ dans le tissu inflammatoire, une réduction indiquant que l'antagoniste agit au niveau d'αEβ7.

9. Procédé selon la revendication 8 dans lequel l'animal a une maladie intestinale inflammatoire établie produite par un réactif haptène.

10. Procédé selon la revendication 9 dans lequel le réactif haptène est un conjugué 2,4,6 trinitrophénol (TNP) d'hémocyanine de patelle (KLH, keyhole limpet hemocyanin).

11. Procédé selon la revendication 10, dans lequel l'animal est une souris.

12. Procédé de criblage à la recherche d'une substance efficace pour prévenir les effets inflammatoires d'αEβ7 dans une maladie intestinale inflammatoire, comprenant les étapes consistant à :
a) doser les cellules de tissu inflammatoire d'un animal non humain qui est sensible à une maladie intestinale inflammatoire et qui a été soumis à un traitement qui provoque une réponse inflammatoire, pour déterminer une quantité de sécrétion de cytokines pro-inflammatoires, de cytokines inflammatoires ou de chimiokines, une absence d'augmentation de la quantité de cytokines pro-inflammatoires, de cytokines inflammatoires ou de chimiokines sécrétées dans les cellules de tissu inflammatoire de l'animal comparativement à une augmentation de la quantité de cytokines pro-inflammatoires, de cytokines inflammatoires ou de chimiokines sécrétée par les cellules de tissu inflammatoire d'un animal de contrôle ayant une maladie intestinale inflammatoire et n'ayant pas reçu la substance indiquant que la substance est efficace pour prévenir la maladie inflammatoire en inhibant l'effet inflammatoire d'αEβ7 dans la maladie intestinale inflammatoire ;
b) évaluer l'effet de l'antagoniste d'αEβ7 pour réduire l'adhésion/la rétention/l'influx de cellules inflammatoires αEβ7+ dans le tissu inflammatoire.

13. Procédé selon la revendication 12, dans lequel l'animal est une souris.

14. Procédé selon la revendication 13, dans lequel le traitement qui provoquera la réponse inflammatoire est l'introduction d'une quantité efficace d'un réactif haptène.

15. Procédé selon la revendication 14, dans lequel le réactif haptène est un conjugué 2,4,6 trinitrophénol (TNP) d'hémocyanine de patelle (KLH, keyhole limpet hemocyanin).

16. Procédé selon la revendication 8 dans lequel la cytokine est l'IFN-γ.

17. Procédé selon la revendication 12 dans lequel la cytokine est l'IFN-γ.
